# EUROPEAN PATENT SPECIFICATION

(11) **EP 2 207 914 B1**
(45) Date of publication and mention of the grant of the patent: **18.04.2012**
(21) Application number: 08807733.4
(22) Date of filing: 19.09.2008
(51) Int. Cl.: C25D 11/16, C25D 5/34, C23C 28/00, A61F 2/30, A61L 27/32, A61C 8/00, A61K 6/00, A61L 27/50, A61K 6/04, A61L 27/06

(54) **METHOD FOR MAXIMISING AND RENDERING UNIFORM THE CONTACT SURFACE ON AN IMPLANT**
VERFAHREN ZUM MAXIMIEREN UND EINHEITLICHMACHEN DER KONTAKTOBERFLÄCHE EINES IMPLANTATS
PROCÉDÉ PERMETTANT DE MAXIMISER ET D'UNIFORMISER LA SURFACE DE CONTACT D'UN IMPLANT

(30) Priority: 21.09.2007 ES 200702492
(43) Date of publication of application: 21.07.2010
(73) Proprietor: Vilardell Purti, S.A., 08251 Santpedor (ES)
(72) Inventor: MESTRE VINARDELL, Aleix, E-08251 Santpedor (ES); CORTINA CAIXACH, Anna, E-08251 Santpedor (ES)
(74) Representative: Ponti Sales, Adelaida
(86) International application number: PCT/IB2008/053818
(87) International publication number: WO 2009/037670

(56) References cited:
- WO-A-02/07792
- WO-A-02/078759
- WO-A-2004/000378
- WO-A-2004/008983
- US-A- 2 928 776
- US-A1- 2007 125 651

## Description

### Field of the invention

The present invention relates to a method for maximising and rendering uniform the contact surface on an implant. The present invention also relates to the implant obtained by this method. In particular, said implant is a dental implant.

### Background

An implant is an appliance, prosthesis or substance that is placed in the body in order to improve some of its functions or for aesthetic purposes. In particular, a dental implant is an element which allows the root of a tooth to be replaced. The implant generally takes the form of an outwardly-threaded cylinder for implantation and stabilisation in the bone tissue, and the material used for manufacturing is usually Ti, Zr, Ta, Nb, Al, V, Mg and alloys thereof.

In order to fit the implant, this is inserted into the jaw or jawbone of the patient by means of a surgical operation. Once the implant has been fixed in position, a pillar is added to form the base for later fitting of the definitive prosthesis. The dimensional requirements and surface finishes of the various parts that make up an implant are, among others, decisive factors in ensuring success.

The fitting of a dental implant offers many advantages when compared to the traditional methods based on bridges and crowns. Among others, the main advantages include the following:
- functional improvements: easier mastication and speech, greater duration and minimum maintenance.
- aesthetic and anatomical improvements: maintenance of the facial outline and prevention of bone loss, increased confidence in dealing with various everyday situations, etc.

The main disadvantage of treatment with implants has historically been the complex process involved and the total time from the start of the treatment through to completion, from the viewpoint of both odontologist and patient.

Initially, the treatment is carried out in two steps. The dentist starts by fitting the implant and then leaving it for a period of between 3 and 6 months, the period considered to be necessary for achieving a full integration between the implant and the bone. During this period the patient wears a provisional prosthesis until, once correct fixing of the implant has been insured, the definitive prosthesis is fitted.

The trend in developing this product and successive improvements thereto has been directed towards reducing this period of time between the start and end of the treatment.

The innovations have focused on achieving surfaces with certain chemical and topographical characteristics that optimise and accelerate the bone integration process. Over the years, various techniques have been developed to confer said characteristics on the implant surface. Among these techniques the more remarkable ones are plasma-spraying of calcium or titanium phosphates, acid etching, passivation, projection of particles and anodic oxidation.

One of the surface characteristics that most directly influences bone cellular response is roughness, while the total dimension of the contact area between the implant and the bone contributes to a better primary stability. It is known that the greater the contact area the greater the primary stability. See, for example, Conrado Aparicio et al., Corrosion behaviour of commercially pure titanium shot blasted with different materials and sizes of shot particles for dental implant applications. Biomaterials 24 (2003) 263-273. Some surface treatments therefore seek to maximise the real surface area of the implants in order to increase the implant/bone contact area.

US Patent 5,478,237 to Ishizawa discloses the disadvantages of the existing surface treatment techniques such as plasma spray, TPS (titanium plasma spray), immersion, etc., and reaches the conclusion of providing a new surface treatment to achieve a titanium implant of complex geometry with a biologically active surface treatment. In said treatment titanium is used as the foundation for creating layers:
- layer formed from the TPS,
- layer provided by acid etching or sand blasting (shot blasting)
- anodic oxidation layer
- layer of Ca and P compounds
- hydrothermal treatment

Said patent also discloses the advantages of anodising: good adhesion of the material and the layer and a uniform treatment even with complex geometries.

US patent 2005/0019365 to Frauchiger discloses the disadvantages of the Ishizawa patent (large number of steps and formation of hydroxyapatite crystals that may be fragile greatly weakening the layer). The process disclosed achieves a surface containing between 1 and 40% by volume of calcium phosphate compounds. Optional pretreatments include a shot blasting and/or acid etching step.

The article J.C. de Vicente, O. Recio, L. Martin-Villa, L. M. Junquera, J.S. López-Arranz:Histomorphometric evaluation of guided bone regeneration around implants with SLA surface: an experimental study in beagle dogs. Int.J.Oral Maxillofacial.S urg.2006;35:1047-1053, discloses a study of implants with SLA surface using shot blasting and an acid etching step, but without anodising. It is inferred then that the shot blasting technique does not have to be combined with anodising.

The article M. Papakyriacou et al.Effects of surface treatments on high cycle corrosion fatigue of metallic implant materials. International Journal of Fatigue 22 (2000) 873-886 discloses a fatigue study wherein implants subjected to shot blasting are used.

None of the techniques known in the art achieves a layer enriched for Ca, P and F with a contact surface increased more than 75% (presence of macroroughness and microporosity) reducing at the same time the number of steps to only one physical treatment and one chemical treatment step, and furthermore providing a more uniform layer.

### Brief description of the figures

Figure 1 pertains to an observation using SEM (Scanning Electron Microscopy) of a polished surface of the alloy Ti₆Al₄V ELI.
Figure 2 pertains to SEM observation of a surface of the alloy Ti₆Al₄V ELI to which a shot blasting treatment with aluminium oxide particles (Al₂O₃) has been applied.
Figure 3 pertains to SEM observation of a surface of the alloy Ti₆Al₄V ELI to which an anodic oxidation treatment has been applied.
Figure 4 pertains to SEM observation of a surface of the alloy Ti₆Al₄V ELI to which a shot blasting treatment with particles of aluminium oxide (Al₂O₃) has been applied, followed by anodic oxidation under the conditions disclosed in the method of the present invention.

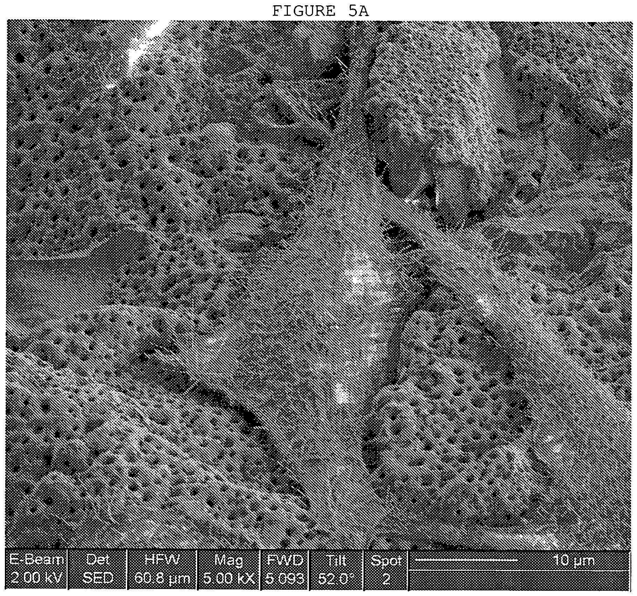

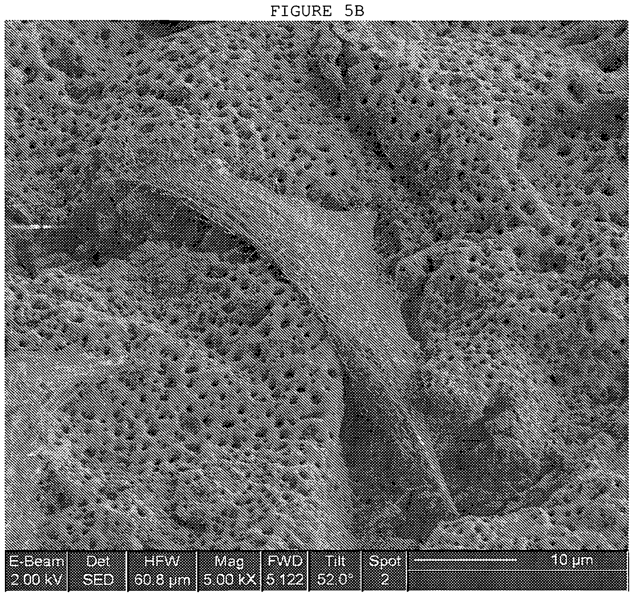

Figures 5A and 5B show electron microscopy images of a culture of osteoblasts on the treated surface (shot blasting + anodic oxidation) after 24 hours of incubation. The presence of cytoplasmatic extensions (filipodes) is observed on the back of the cell.

### Brief description of the invention

The present invention relates to a method for maximising and rendering uniform the contact surface on an implant. In particular, said implant is a dental implant.

A first object of the present invention is a method for maximising and rendering uniform the contact surface on an implant by using a physical treatment (shot blasting) and a chemical treatment (anodic oxidation). In particular, said implant is a dental implant.

A second object of the present invention is an implant obtained by the method according to the first object of the invention. In particular, said implant is a dental implant.

### Description of the invention

A first object of the present invention relates to a method for maximising and rendering uniform the contact surface on an implant, in particular a dental implant, which includes the steps of:
A) Physical treatment by shot blasting onto the implant using particles selected from the group consisting of aluminium oxide, silicon carbide, titanium oxide, zirconium oxide, microbeads of glass, with an optimum size to ensure that the entire geometry of the implant is uniformly treated at a P between 1 and 8 bars (1x10⁵ and 8x10⁵ Pa), preferably between 3 and 5 bars (3x10⁵ and 5x10⁵ Pa), for 30-40 seconds;
B) Chemical treatment by anodic oxidation comprising:
   1) preparation of an electrolyte formed by soluble compounds of Ca, P and F, at least one complexing agent and an acid or base solution in deionised water;
   2) addition of at least one surfactant
   3) application of the following oxidation parameters to the tank with electrolyte, wherein the implant will be the anode and the cathode will be made up of a metallic mesh preferably manufactured from titanium, platinum or stainless steel
      - constant current density of approximately 0.75 mA/mm²
      - potential: rising between 0 and 200 V
      - time: 40-90 seconds.

Although not specifically disclosed in the method described above, it will be obvious to a skilled person in the art that cleaning steps well-known in the art are carried out both after the shot blasting treatment and before and after the chemical treatment. Likewise, preparing the electrolyte will be obvious to a skilled person in the art that shaking is carried out for the time necessary to achieve an homogeneous electrolyte (approximately 24 hours).

The materials used for said implant include those usual in the field, preferably Ti, Zr, Ta, Nb, Al, V, Mg, and alloys thereof.

In step A it is preferable to use particles of aluminium oxide between 212 and 300 µm, while the time necessary for this step is preferably from 30 to 40 seconds.

In step (B1) the Ca compound of the electrolyte includes, without limitation, calcium acetate, calcium glycerophosphate and calcium citrate. In a more preferred embodiment said compound of Ca is calcium acetate. The concentration of said compound of Ca in the electrolyte is up to a maximum of 63.8 g/l.

In step (B1) the P compound of the electrolyte includes, without limitation, calcium bis(dihydrogen phosphate), phosphoric acid, calcium glycerophosphate and sodium glycerophosphate. In a more preferred embodiment said compound of P is calcium bis(dihydrogen phosphate). The concentration of said compound of P in the electrolyte is up to a maximum of 31.5 g/l.

In step (B1) the F compound of the electrolyte includes, without limitation, sodium fluoride and ammonium fluoride. The concentration of said F compounds in the electrolyte is up to a maximum of 30 g/l.

In step (B1) the complexing agent of the electrolyte includes, without limitation, inorganic carboxylic acids (citric acid, tartaric acid, nitriloacetic acid, EDTA, CDTA, DTPA, 2-hydroxy-ethylendiamintriacetic acid, TTHA), ketones (diketones, polyketones), organophosphoric acids or organophosphates (with more than two phosphate groups), organophosphoric acids or organophosphonates (with more than two phosphonate groups), organophosphorous acids and/or organophosphites (with more than two phosphite groups). In a more preferred embodiment said complexing agent is EDTA. The concentration of said complexing agent in the electrolyte is up to a maximum of 50 g/l.

In step (B1) an acid or base solution is used, preferably a base solution. A solution for obtaining a basic pH includes, without limitation, a solution of alkaline and/or alkaline earth metal hydroxide, preferably sodium. A solution for obtaining an acid pH includes, without limitation, a solution of sulphuric acid, nitric acid, hydrochloric acid and/or phosphoric acid.

In step (B2) the surfactant includes sodium and/or potassium salts of straight-chain fatty acids, straight-chain alkylbenzenosulphate, paraffin sulphonates, α-olefin sulphonates, dialkyl sulphosuccinates, alkyl sulphates, alkyl polyether sulphates, alkyl phosphates, long-chain alkylbenzene sulphonates, preferably containing from 8 to 20 atoms of C, fatty amines and salts thereof, quaternary ammonium salts, polyethoxylated fatty amines, polyethoxylated alkylphenols, polyethoxylated fatty alcohols, polyethoxylated fatty acids and alkanolamides and/or alkanolamine condensates. In a more preferred embodiment said surfactant is sodium lauryl sulphate. The concentration of said surfactant of step (B2) of the method is up to a maximum of 50 g/l.

The control parameters of the oxidation of step (B3) include current density, potential, time and temperature.

The current intensity applied depends on the result of combining a constant current density, implant area and the factor indicating the surface increase achieved in the physical treatment (shot blasting), obtained using an optical interferometer. The present inventors have observed that the optimum current density is approximately 0.75 mA/mm². With lower current densities the process would need more time to reach a potential difference of 140 V and the process would not be industrially viable. With higher densities the properties of the layer would not be the same, as the growth of the oxide layer would not be homogeneous.

The potential used starts at 0 and rises freely up to the limit set over the course of the process. The work is preferably carried out within a range between 100 and 170 V, more preferably between 130 and 140 V.

The current application time is usually variable depending on the surface to be anodised, but is preferably between 40 and 90 seconds.

The temperature used is that normal in this kind of process, i.e. room temperature.

Surprisingly, the present inventors have observed that the use of said method does not require the application of any type of pre-treatment based on an etching acid to achieve roughness, since in such cases there exists a high risk of hydrides being incorporated on the surface and thus rendering the body of the implant fragile.

Additionally, the results of said method achieve an increase of over 75% in the total contact area of the implant in relation to its initial value, while permitting continuity of the already-known advantages of anodic oxidation, such as the incorporation of chemical elements (for example Ca, P, F) by attempting to achieve the maximum contact and integration with the bone. Topographically, an implant is obtained with a macroroughness and microporosity that seeks to be biomimetic with its biological environment. A single chemical process is used to create a uniform microporosity over the entire geometry of the implant, achieving a Ca/P ratio similar to that of hydroxyapatite.

As a novel feature a surfactant has been incorporated into the anodic oxidation process in order to reduce the size of the bubbles formed on the surface of the metal during the process and thereby reduce the negative impact such oxygen bubbles can have on the surface treatment, both aesthetically and functionally. Unlike what could happen with a smooth surface, a rough surface increases the possibility of the bubbles adhering more easily on the pores or rough zones. The surfactant therefore allows a correct homogenisation of the treatment over the entire treated surface avoiding an excessive oxidation at certain zones that could negatively affect the properties of the obtained coating (for example, chipping and fragility of the obtained layer).

Another novel feature is the use of a constant current density suited to the dimension of the implant in order to optimise the topography of the surface. If it were carried out at a constant potential and varying the current density, as it is usually done in the art, it would be more difficult to control the process, since it should be determined which intensity is necessary for stopping the process, which would be different depending on the number of implants to be anodised. Additionally, the growth of the layer would not be exactly equal since at the beginning of the experiment there would be too much current, but that current which would decrease over time giving rising to a certain heterogeneity on the surface. The use of a constant current density therefore achieves more controlled and uniform growth of the layer.

Table 1 below shows the results of evolution of the surface-increase microtopographic parameter (%) as a function of the treatments applied: shot blasting, anodic oxidation and shot blasting + anodic oxidation on a polished Ti₆Al₄V surface.

**Table 1**

| Comparison of implants | |
|---|---|
| | Increase of surface (%) |
| Shot Blasting | 29 |
| Anodic oxidation | 29 |
| Shot Blasting + Anodic oxidation | 76 |

From Table 1, an increase of surface when using the treatment of the present invention can be observed clearly. The techniques used for calculating this microtopographical parameter are based on the Contactless Profilometry described by Buddy D. Ratner et al., Biomaterials Science (2nd edition) chapter 2.15, Elsevier Academic Press.

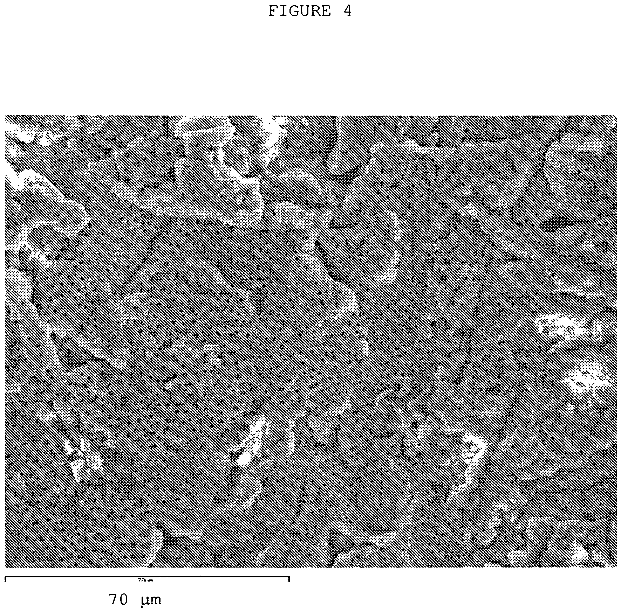

In relation to the figures illustrating the present invention, Figure 4 clearly shows that combining the processes of shot blasting + anodic oxidation provides a surface characterised by macroroughness ("level differences") and microporosity (pores resembling craters in appearance).

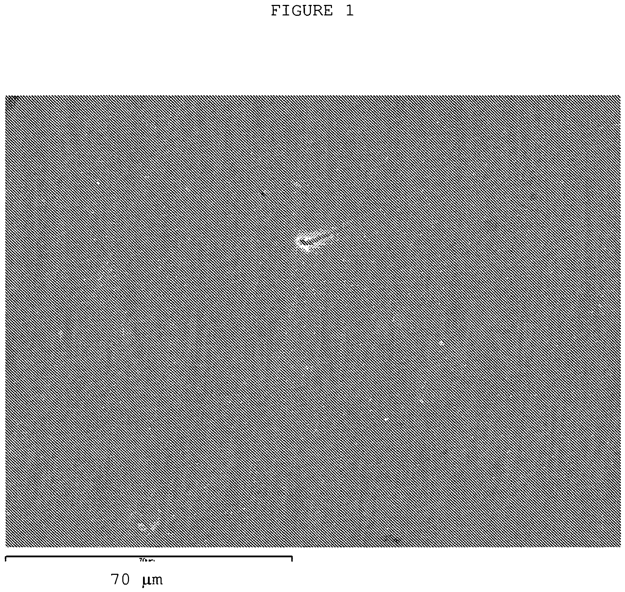

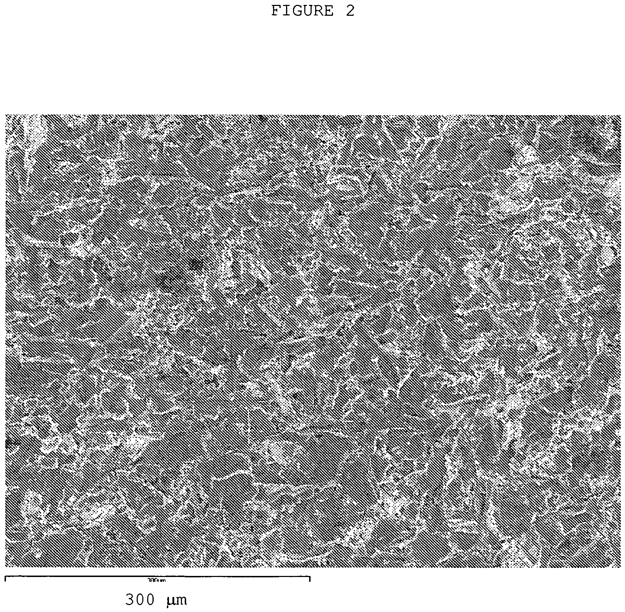

If compared with the surface of Figure 2 (shot-blasting only) it is observed that this one also shows macroroughness compared with the totally smooth surface (Figure 1), having a topography made up of very pronounced edges, although there is no formation of pores (craters).

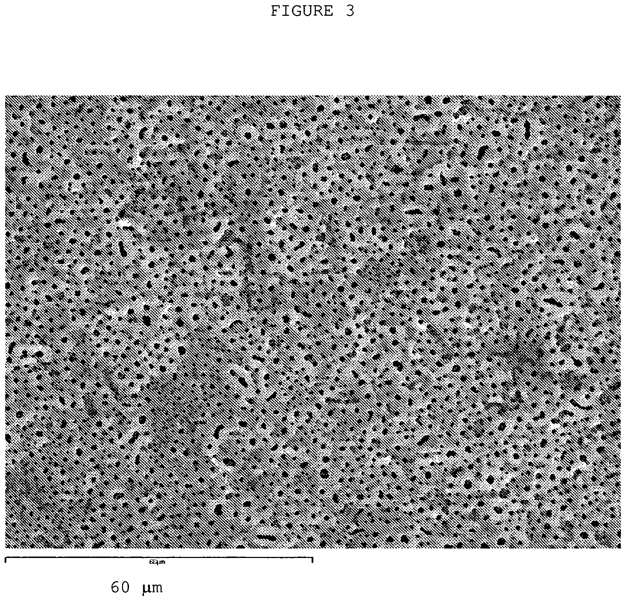

When compared with the surface of Figure 3 (only anodisation) the presence of pores can be observed, but the level differences are not so clear (topography with more rounded edges) and are like those observed on the surface treated only with shot-blasting or with both treatments combined.

It is therefore clear that both treatments used separately do increase the contact surface, though in a proportion and characteristics which are different from when the two are combined, since the increase of surface area is then more marked and the topography shows both macroroughness and microporosity, aspects significantly important in the adhesion of the cells and in subsequent osseointegration.

A second object of the present invention relates to the implant obtained by the method described in the present invention. In particular, said implant is a dental implant.

By way of non-restrictive illustration there follows a description of an example of an embodiment of the present invention:

### Example 1

A shot blasting treatment with particles of aluminium oxide (Al₂O₃) was carried out for 20 seconds at a pressure of 3 bars on a 10 mm rotating base. In a parallel way, an electrolyte with the following composition was prepared:

| CHEMICAL COMPOUND | CONCENTRATION |
|---|---|
| Calcium hydrogen phosphate [Ca (H₂PO₄)] | 10 g/l |
| Calcium acetate [Ca(COOCCH₃)₂] | 6 g/l |
| Na₂(EDTA) | 22 g/l |
| NaF | 30 g/l |
| Sodium lauryl sulphate (C₁₂H₂₅NaO₄S) | 2 g/l |
| Deionized water | q.s. up to 1 litre |

After placing the implant in the anodic oxidation tank together with the electrolyte and a titanium mesh, the following oxidation parameters were set:
- Intensity: 0,0736 A
- Potential rising up to 170 V
- Temperature of 25°C

Finally, the rotating base was cleaned with deionised water and ultrasound.

## Claims

1. Method for maximising and rendering uniform the contact surface of an implant, comprising the steps of:
A) Physical treatment by shot blasting onto the implant using particles selected from the group consisting of aluminium oxide, silicon carbide, titanium oxide, zirconium oxide, microbeads of glass, with an optimum size to ensure that the entire geometry of the implant is uniformly treated at a P between 1 and 8 bars (1 x 10⁵ and 8 x 10⁵ Pa), preferably between 3 and 5 bars (3 x 10⁵ and 5 x 10⁵ Pa), for 30-40 seconds;
B) Chemical treatment by anodic oxidation comprising:
1) preparation of an electrolyte formed by soluble compounds of Ca, P and F, at least one complexing agent and an acid or base solution in deionised water;
2) addition of at least one surfactant;
3) application of the following oxidation parameters to the tank with electrolyte, wherein the implant will be the anode and the cathode will be made up of a metallic mesh:
- a constant current density of approximately 0.75 mA/mm²
- potential: rising between 0 and 200 V
- time:40-90 seconds.

2. Method according to claim 1, wherein the particle size of the aluminium oxide is in the range from 212 to 300 µm.

3. Method according to claim 1, wherein the surfactant used in step B2) comprises sodium and/or potassium salts of straight-chain fatty acids, straight-chain alkylbenzenosulphate, paraffin sulphonates, α-olefin sulphonates, dialkyl sulphosuccinates, alkyl sulphates, alkyl polyether sulphates, alkyl phosphates, long-chain alkylbenzene sulphonates, preferably containing from 8 to 20 atoms of C, fatty amines and salts thereof, quaternary ammonium salts, polyethoxylated fatty amines, polyethoxylated alkylphenols, polyethoxylated fatty alcohols, polyethoxylated fatty acids and alkanolamides and/or alkanolamine condensates.

4. Method according to claim 3, wherein the surfactant used in step B2) is sodium lauryl sulphate.

5. Method according to claim 1, wherein the Ca compound of the electrolyte comprises calcium acetate, calcium glycerophosphate and/or calcium citrate.

6. Method according to claim 5, wherein said Ca compound of the electrolyte is calcium acetate.

7. Method according to claim 1, wherein the P compound of the electrolyte comprises calcium bis(dihydrogen phosphate), phosphoric acid, calcium glycerophosphate and/or sodium glycerophosphate.

8. Method according to claim 7, wherein said P compound of the electrolyte is calcium bis(dihydrogen phosphate).

9. Method according to claim 1, wherein the F compound of the electrolyte comprises sodium fluoride and/or ammonium fluoride.

10. Method according to claim 1, wherein the complexing agent of the electrolyte comprises inorganic carboxylic acids (citric acid, tartaric acid, nitriloacetic acid, EDTA, CDTA, DTPA, 2-hydroxy-ethylendiamintriacetic acid, TTHA), ketones (diketones, polyketones), organophosphoric acids or organophosphates (with more than two phosphate groups), organophosphoric acids or organophosphonates (with more than two phosphonate groups), organophosphorous acids and/or organophosphites (with more than two phosphite groups).

11. Method according to claim 9, wherein said complexing agent of the electrolyte is EDTA.

12. Method according to claim 1, wherein the base solution comprises a solution of alkaline and/or alkaline earth metal hydroxide.

13. Method according to claim 11, wherein the base solution is sodium hydroxide.

14. Method according to claim 1, wherein the acid solution comprises a solution of sulphuric acid, nitric acid, hydrochloric acid and/or phosphoric acid.

15. Method according to claim 1, wherein the metallic mesh is manufactured from titanium, platinum or stainless steel.

16. Method according to any of the preceding claims, wherein the implant is a dental implant.

17. Method according to any of the preceding claims, wherein the material of the implant is selected from the group consisting of Ti, Zr, Ta, Nb, Al, V, Mg and alloys thereof.

## Patentansprüche

1. Verfahren zum Maximieren und Einheitlichmachen der Kontaktoberfläche eines Implantats, das folgende Schritte aufweist:
A) Physische Behandlung durch Sandstrahlen auf das Implantat unter Verwendung von Partikeln, ausgewählt aus der Gruppe, die besteht aus Aluminiumoxyd, Siliciumkarbid, Titanoxyd, Zirkonoxyd, Mikrokügelchen von Glas, mit einer optimalen Größe, um sicherzustellen, dass die gesamte Geometrie des Implantats gleichmäßig bei einem Druck zwischen 1 und 8 bar (1 x 10⁵ und 8 x 10⁵ Pa), bevorzugt zwischen 3 und 5 bar (3 x 10⁵ und 5x 10⁵ Pa) für 30 bis 40 Sekunden behandelt wird;
B) Chemisches Behandeln durch anodische Oxidation aufweisend:
1) Zubereiten eines Elektrolyten gebildet aus löslichen Komponenten von Kalzium, Phosphor und Fluor, wenigstens einem komplexen Zusatzstoff und einer sauren oder basischen Lösung in deionisiertem Wasser;
2) Addition von wenigstens einem Tensid;
3) Aufbringen der nachfolgenden Oxidationsparameter zu dem Behältnis mit Elektrolyt, wobei das Implantat die Anode sein wird und die Kathode aus einem Metallgitter gebildet wird:
- einer konstanten Stromdichte von ungefähr 0,75 mA/mm²
- Potential: steigend von 0 bis 200 V
- Zeit: 40 bis 90 Sekunden.

2. Verfahren nach Anspruch 1, wobei die Partikelgröße des Aluminiumoxyds im Bereich von 212 bis 303 µm liegt.

3. Verfahren nach Anspruch 2, wobei das Tensid, das im Schritt B2) verwendet wird, Natrium- und/Kaliumsalze von geradkettigen Fettsäuren, geradkettigen Alkylbenzenosulfat, Paraffinsulphonate, α-Olefinsulphonate, Dialkylsuphosukzinate, Alkylsylphonat, Alkylpolyethersulfat, Alkylphosphat, langkettiges Alkylbenzensulfphonat, vorzugsweise enthaltend 8 bis 20 Atome von Kohlenstoff, fette Amine und Salze davon, quartäre Ammoniumsaltze, polyethoxylatierte fette Amine, polyethoxylate Alkylphenole, polyethoxylatierte fette Alkohole, polyethoxylatierte Fettsäuren und Alkanolamide und/oder Alkanolamin-Kondensate.

4. Verfahren nach Anspruch 3, wobei das im Schritt B2) verwendete Tensid Natriumlaurylsulfat ist.

5. Verfahren nach Anspruch 1, wobei die Kalziumkomponente des Elektrolyten Kalziumacetat, Kalziumglycerophosphat und/oder Kalziumzitrat aufweist.

6. Verfahren nach Anspruch 5, wobei die Kalziumkomponente des Elektrolyten Kalziumacetat ist.

7. Verfahren nach Anspruch 1, wobei die P-Komponente des Elektrolyten ein Kalziumbis(Dihydrogenphosphat), Phosphorsäure, Kalziumglyzerophosphat und/oder Natriumglycerophosphat aufweist.

8. Verfahren nach Anspruch 7, wobei die P-Komponente des Elektrolyten Kalziumbis(Dihydrogenphosphat) ist.

9. Verfahren nach Anspruch 1, wobei die F-Komponente des Elektrolyten Natriumfluorid und/oder Ammoniumfluorid aufweist.

10. Verfahren nach Anspuch 1, wobei der Komplexbildner des Elektrolyten anorganische karboxylische Säuren (Zitronensäure, Weinsäure, Nitrilacetsäure, EDTA, CDTA, DTPA, 2-hydroxy-ethylendiamint-Essigsäure, TTHA), Ketone (Diketone, Polykethone), organphosphore Säuren oder Organphosphate (mit mehr als zwei Phosphatgruppen), Organphosphorsäuren oder Organphosphonate (mit mehr als zwei Phosphonatgruppen), organphosphorische Säuren und/oder Organphosphite (mit mehr als zwei Phosphitgruppen) aufweist.

11. Verfahren nach Anspruch 9, wobei der Komplexbildner des Elektrolyten EDTA ist.

12. Verfahren nach Anspruch 1, wobei die Grundlösung eine Lösung von Alkalin und/oder Alkalinerdmetallhydroxyd aufweist.

13. Verfahren nach Anspruch 11, wobei die Grundlösung Natriumhydroxyd ist.

14. Verfahren nach Anspruch 1, wobei die Säurelösung eine Lösung von Schwefelsäure, Salpetersäure, Salzsäure und/oder Phosphorsäure aufweist.

15. Verfahren nach Anspruch 1, wobei das Metallgitter hergestellt wird aus Titan, Platin oder rostfreiem Stahl.

16. Verfahren nach einem der vorhergehenden Ansprüche, wobei das Implantat ein Zahnimplantat ist.

17. Verfahren nach einem der vorhergehenden Ansprüche, wobei das Material des Implantats ausgewählt ist aus der Gruppe bestehend aus Ti, Zr, Ta, Nb, Al, V, Mg und Legierungen davon.

## Revendications

1. Procédé pour maximiser et rendre uniforme la surface de contact d'un implant, comprenant les étapes de :
A) Traitement physique par grenaillage sur l'implant en utilisant des particules choisies parmi le groupe composé d'oxyde d'aluminium, carbure de silicium, oxyde de titane, oxyde de zirconium, microbilles de verre, avec une taille optimale pour garantir que toute la géométrie de l'implant est traitée uniformément à une P entre 1 et 8 bars (1 x 10⁵ et 8 x 10⁵ Pa), de préférence entre 3 et 5 bars (3 x 10⁵ et 5 x 10⁵ Pa), pendant 30-40 secondes ;
B) Traitement chimique par anodisation comprenant :
1) la préparation d'un électrolyte formé par des composés solubles de Ca, P et F, au moins un agent complexant et une solution acide ou basique dans de l'eau désionisée ;
2) l'ajout d'au moins un tensio-actif ;
3) l'application des paramètres d'oxydation suivants au réservoir avec l'électrolyte, dans lequel l'implant sera l'anode et la cathode sera constituée d'une maille métallique :
- une densité de courant constante d'environ 0,75 mA/mm²
- potentiel : croissant entre 0 et 200 V
- durée : 40-90 secondes.

2. Procédé selon la revendication 1, dans lequel la taille des particules de l'oxyde d'aluminium se situe dans la plage allant de 212 à 300 µm.

3. Procédé selon la revendication 1, dans lequel le tensio-actif utilisé à l'étape B2) comprend des sels de sodium et/ou potassium d'acides gras à chaîne linéaire, alkylbenzénosulfate à chaîne linéaire, paraffine sulfonates, α-oléfine sulfonates, dialkylsulfosuccinates, alkylsulfates, alkylpolyéthersulfates, alkylphosphates, alkylbenzène sulfonates à chaîne longue, contenant de préférence 8 à 20 atomes de C, des amines grasses et leurs sels, des sels d'ammonium quaternaire, des amines grasses polyéthoxylées, des alkylphénols polyéthoxylés, des alcools gras polyéthoxylés, des acides gras polyéthoxylés et des alcanolamides et/ou condensats d'alcanolamine.

4. Procédé selon la revendication 3, dans lequel le tensio-actif utilisé à l'étape B2) est du laurylsulfate de sodium.

5. Procédé selon la revendication 1, dans lequel le composé Ca de l'électrolyte comprend de l'acétate de calcium, du glycérophosphate de calcium et/ou du citrate de calcium.

6. Procédé selon la revendication 5, dans lequel ledit composé Ca de l'électrolyte est de l'acétate de calcium.

7. Procédé selon la revendication 1, dans lequel le composé P de l'électrolyte comprend du bis(dihydrogènephosphate) de calcium, de l'acide phosphorique, du glycérophosphate de calcium et/ou du glycérophosphate de sodium.

8. Procédé selon la revendication 7, dans lequel ledit composé P de l'électrolyte est du bis(dihydrogènephosphate) de calcium.

9. Procédé selon la revendication 1, dans lequel le composé F de l'électrolyte comprend du fluorure de sodium et/ou du fluorure d'ammonium.

10. Procédé selon la revendication 1, dans lequel l'agent complexant de l'électrolyte comprend des acides carboxyliques inorganiques (acide citrique, acide tartrique, acide nitriloacétique, EDTA, CDTA, DTPA, acide 2-hydroxyéthylènediaminetriacétique, TTHA), des cétones (dicétones, polycétones), des acides organophosphoriques ou des organophosphates (avec plus de deux groupes phosphate), des acides organophosphoriques ou des organophosphonates (avec plus de deux groupes phosphonate), des acides organophosphoreux et/ou des organophosphites (avec plus de deux groupes phosphites).

11. Procédé selon la revendication 9, dans lequel ledit agent complexant de l'électrolyte est de l'EDTA.

12. Procédé selon la revendication 1, dans lequel la solution de base comprend une solution d'hydroxyde de métaux alcalins et/ou alcalino-terreux.

13. Procédé selon la revendication 11, dans lequel la solution de base est de l'hydroxyde de sodium.

14. Procédé selon la revendication 1, dans lequel la solution acide comprend une solution d'acide sulfurique, acide nitrique, acide chlorhydrique et/ou acide phosphorique.

15. Procédé selon la revendication 1, dans lequel la maille métallique est fabriquée à partir de titane, de platine ou d'acier inoxydable.

16. Procédé selon l'une quelconque des revendications précédentes, dans lequel l'implant est un implant dentaire.

17. Procédé selon l'une quelconque des revendications précédentes, dans lequel le matériau de l'implant est choisi parmi le groupe composé de Ti, Zr, Ta, Nb, AI, V, Mg et leurs alliages.
